# EUROPEAN PATENT SPECIFICATION

(11) **EP 3 085 789 B1**
(45) Date of publication and mention of the grant of the patent: **15.05.2019**
(21) Application number: 14871485.0
(22) Date of filing: 04.11.2014
(51) Int. Cl.: C12P 19/24, C12N 9/26, A23L 21/25

(54) **A PROCESS OF PRODUCING A HONEY CONCENTRATE**
VERFAHREN ZUR HERSTELLUNG EINES HONIGKONZENTRATS
PROCÉDÉ DE PRODUCTION D' UN CONCENTRÉ DE MIEL

(30) Priority: 16.12.2013 UZ 1300524
(43) Date of publication of application: 26.10.2016
(73) Proprietor: Mkrtchyan, Ovik Leonardovich, Tashkent 100052 (UZ)
(72) Inventor: Mkrtchyan, Ovik Leonardovich, Tashkent 100052 (UZ)
(74) Representative: KATZAROV S.A.
(86) International application number: PCT/UZ2014/000004
(87) International publication number: WO 2015/095898

(56) References cited:
- RU-C1- 2 323 965
- RU-C2- 2 465 784
- 'Podkormka. Pchelovodstvo. Forum pchelovodov MFU' 18 November 2011, page 1, XP055359105
- KHORN X.: 'Vse o mede: proizvodstvo, poluchenie, ekologicheskaia chistota i sbyt. Sostav meda: uglevody, voda, proteiny.' M, ACT: ASTREL 2007, pages 1 - 6, XP008184014
- 'Kak pchely delaiut med, sozrevanie meda' 02 December 2011, page 1., XP008183883 Retrieved from the Internet: <URL:http://medokmed.ra/tag/sozrevanie-meda >
- DUBTSOVA E. A.: 'Med, ego sostav, svoistva i vliianie na biologicheskii vozrast' KLINICHESKAIA GERONTOLOGIIA 2008, pages 38 - 39, XP055351615
- KOMLATSKII V. I. ET AL.: 'Pchelovodstvo , Rostov-na-Donu, izd-vo' FENIKS 2009, pages 196 - 197,227, XP008183885

## Description

The invention relates to the fields of biotechnology and medicine, more particularly to processes of producing a honey concentrate, and it may be useful in medicine, agriculture, pharmaceutics, cosmetics.

One of the most famous high-molecular naturally occurring polymers is honey. About 25 sugars are honey constituents. The main honey sugars are monosaccharides: glucose or grape sugar (27 - 36%) and fructose or fruit sugar (33 - 42%). In addition, essential honey components are enzymes. Over 15 enzymes had been found to be honey components. Among them, invertase and diastase are the main enzymes present in honey.

Honey is indispensable product which is used in naturally occurring form. It is a component of many medicaments and may be useful for making medicinal preparations, rational and dietic food, cosmetic products.

Natural bee-produced honey comprises valuable mineral substances, microelements, vitamins, enzymes, biologically active substances exhibiting bactericidal properties.

Nutritive properties of honey stem from the point that componentry of such product includes carbohydrates, mostly fructose and glucose, vitamins, enzymes, mineral and some other substances that take an active part in the entire metabolism which occurs in a living organism. However, as honey relates to high molecular weight naturally occurring compounds, a problem of attaining the increased assimilability of honey in a living organism has been remaining unsolved.

It has been known that enzymatic hydrolysis technique is a changer of the high-molecular structure of naturally occurring polymers.

The prior art knows a process of isolating low-molecular peptides useful for preparing biologically active supplements (BAS). The known process provides for preparation of an enzymatic hydrolyzate to be made of colostric casein-serum mixture using pancreatin. The hydrolyzate thus prepared is exposed to adding sephadex G-25 in a proportion of 300 g sephadex per 1.0 liter of the enzymatic hydrolyzate. The resultant mixture is held for 10 minutes of residence time before follow-up centrifugation. The swollen gel is subjected to elution with distilled water and exposed to centrifugation again. The liquid fractions thus produced are sterilized by filtration using microporous filters having pore diameter of 0.2 µmicrons. The known invention enables to broaden the raw material base for production of lo-molecular peptides [Specification describing the patent RU 2416243, IPC A23J 1/20, published on 20.04.2011]. A process for producing an inverted syrup for bees using honey enzymes ("Podkormka. Pchelovodstvo. Forum pchelovodov MFU", 18-11-2011, page 1).

In terms of essential features, a process which may be regarded to be the closest to the known one is a process of producing an enzymatic hydrolyzate of chicken embryonic tissues for the preparation of protective media in the manufacture of dry viral vaccines, comprising the steps of fining chicken embryos, adding pancreatin, allowing a hydrolysis to occur and assessing a quality of the hydrolyzate thus produced, *characterized in that* in order to enhance quality of a target product prior to the step of fining the chicken embryos are separated from egg shell and yolk sac to prepare a raw material which is further exposed to addition of distilled water in weight proportion of 1 : 1 - 1.5, then the chicken embryos are subjected to fining to produce a homogenate which is further exposed to addition of pancreatin in weight proportion of 240 - 260 : 1, while the hydrolysis is performed at 37 - 39°C and under continuous stirring for 18 - 29 hours [Specification describing the patent RU 1277457, IPC A61K 37/02, C12N 1/00, B01F 17/30, published on 15.10.1994].

However, the known process does not include use, during the enzymatic hydrolysis, of a substrate comprised of native enzymes, while providing for optimal conditions for a progress of enzyme-substrate reaction.

A basis of the invention rests on an objective of providing a novel process of producing a honey concentrate by means of autofermentation, while providing for optimal conditions for a progress of enzyme-substrate reaction.

The objective stated above is gained owing to the point that a process of producing a honey concentrate, comprising a preparatory stage with an addition of distilled water and homogenate heating, performing hydrolysis while stirring continuously and assessing a quality of a hydrolyzate thus produced, wherein *according to the invention* honey is exposed to an addition of 80% of purified water as compared to a total volume of water to be added, a temperature is raised up to 35 - 40°C, and a pH of the homogenate is adjusted up to 6.0, then the temperature is further raised under intensive stirring and pH = 6.0 to make 45°C, a pH value is adjusted every 10 minutes for a period of 50 - 60 minutes in order to achieve a stabilized pH value within a range of 4.5 - 5.0, then a resultant hydrolyzate is cooled down to room temperature, exposed to stirring, subjected to addition of ethanol used as a stabilizer in an amount of 2% of a total volume of the resultant hydrolyzate, the remaining water is subsequently added, the resultant hydrolyzate is stirred again, settled for 60 minutes to produce a honey concentrate which is subsequently filtered, poured into containers and autoclaved at 110°C.

The core of claimed invention is that autofermentation enables to produce a solution of honey, which is free of enzymes and polysugars. The claimed process provides for production of a honey concentrate when necessary autofermentation conditions, namely a cleavage of honey via its native enzymes, are ensured. In the claimed process, saccharose and invertase make an enzyme - substrate pair. Invertase cleaves saccharose to produce monosugars such as glucose and fructose. During autofermentation, a pH value as an acidity parameter of the medium is maintained at 4.5. The honey concentrate is heated up to 40 - 45°C. Autofermentation is regarded finished within 50 - 60 minutes.

The claimed process is illustrated using the following example.

In order to realize the claimed process, the following pieces of equipment have been used:

| m | Piece of equipment | Manufacturer, country | Year of manufacture |
|---|---|---|---|
| 1. | pH meter, conductometer | Mettler Toledo, Switzerland | 2011 |
| 2. | Densimeter | Mettler Toledo, Switzerland | 2011 |
| 3. | Magnetic stirrer with a heater | Ekros-ES-6120, Russia | 2010 |
| 4. | Desktop electronic balance | ST-6000, Shinko, China | 2010 |
| 5. | Analytical balance | Ohaus-200, China | 2011 |

### Stage I. Preparatory phase

A pre-weighted amount of natural honey is placed into a 25 liters stainless steel vessel, which was cleaned and washed in advance. Further, purified water complying with national standard FS 42 Uz - 0511 is added slowly to the vessel, the amount of said purified water being about 80% of a total amount of water to be added. The vessel is installed on Ekros-ES-6120 magnetic stirrer with a heater. The solution is stirred slowly at 600 rpm and 35 - 40°C in order to produce a homogeneous mixture. Afterwards, a pH value of solution is adjusted by means of addition of 0.1M NaOH solution to 6.0, i.e. to optimal value which ensures that fermentation of saccharose by invertase is most active.

### Stage II. Autofermentation

On autofermentation phase, rotational speed of the stirrer, solution temperature, pH value are increased up to 1,000 rpm, 45°C, pH = 6.0, respectively, and starting time of autofermentation is noted. The pH value of solution is adjusted each 10 minutes. Autofermentation is finished when pH of the solution becomes stabilized within a range of 4.5 - 5.0. Autofermentation phase duration is 50 - 60 minutes.

### Stage III. Stabilization

The solution is cooled down to room temperature, stirred, and subjected to addition of ethanol used in an amount of 2% of a total volume. Further, a remaining portion of purified water is added to make the required volume of solution. The solution is stirred carefully and allowed to settle for 60 minutes. At last, resultant honey concentrate is filtered, poured into handy containers, and autoclaved at 110°C.

The honey concentrate has been produced with the following organoleptic, physical and chemical properties as shown in Table 1.

**Table 1**

| Parameter | Product performance |
|---|---|
| 1. Outer appearance and colour | Opaque liquid, lemon or yellow in colour |
| 2. Odour | Characteristic odour of honey |
| 3. pH of concentrate at 20°C | from 3.0 to 6.0 |
| 4. Authenticity | to be confirmed |
| 5. Impurity amount | 30 (0.003) |
| Oxymethylfurfurol in 1 ml of concentrate, mkg (%), at most | |
| 6. Density g/cm³ | from 1.110 to 1.400 |
| 7. Acidity (0.1 mol/l of NaOH solution per 100 g of product, % | from 1.0 to 2.5 |
| 8. Total amount of sugars (fructose and glucose) per 1 ml, g | from 0.1930 to 0.36540 |

Microbiological purity. No presence of *Enterobacteriaceae*, *Pseudomonas aeruginosa*, *Staphylococcus areus* family bacteria is allowed in the concentrate. A total quantity of aerobic bacteria and fungi (cumulatively) per 1 g of the product is allowed to be not more than 10².

Use of the claimed process enables to achieve a full fermentation of saccharose and honey starch, thus leading to greater bioavailability of honey with no changes of quality characteristics.

Use of the claimed process allows to produce a substance of honey concentrate, which is able to broaden substantially the possibilities of using honey in pharmacological and cosmetic industries.

## Claims

1. A process of producing a honey concentrate, comprising a preparatory stage with an addition of distilled water and homogenate heating, performing hydrolysis while stirring continuously and assessing a quality of a hydrolyzate thus produced, ***characterized in that*** honey is exposed to an addition of 80% of purified water as compared to a total volume of water to be added, a temperature is raised up to 35 - 40°C, and a pH value of the homogenate is adjusted up to 6.0, then the temperature is further raised under intensive stirring and pH = 6.0 to make 45°C, a pH value is adjusted every 10 minutes for a period of 50 - 60 minutes in order to achieve a stabilized pH value within a range of 4.5 - 5.0, then a resultant hydrolyzate is cooled down to room temperature, exposed to stirring, subjected to an addition of ethanol used as a stabilizer in an amount of 2% of a total volume of the resultant hydrolyzate, the remaining water is subsequently added, the resultant hydrolyzate is stirred again, settled for 60 minutes to produce a honey concentrate which is subsequently filtered, poured into containers and autoclaved at 110°C.

## Patentansprüche

1. Verfahren zur Herstellung eines Honigkonzentrats, umfassend eine Vorbereitungsphase mit Zugabe von destilliertem Wasser und Homogenisat-Erhitzen, wobei unter kontinuierlichem Rühren eine Hydrolyse durchgeführt und eine Qualität eines so hergestellten Hydrolysats beurteilt wird, ***dadurch gekennzeichnet, dass*** Honig einer Zugabe von 80 % gereinigtem Wasser, relativ zu einem Gesamtvolumen an Wasser, das zugegeben werden soll, ausgesetzt wird, die Temperatur auf 35 bis 40 °C erhöht wird und ein pH-Wert des Homogenats auf 6,0 eingestellt wird, wobei die Temperatur dann unter intensivem Rühren und bei einem pH-Wert von 6,0 weiter bis auf 45 °C erhöht wird, über einen Zeitraum von 50 bis 60 Minuten ein pH-Wert alle 10 Minuten eingestellt wird, um einen stabilisierten pH-Wert innerhalb eines Bereichs von 4,5 bis 5,0 zu erreichen, wobei ein resultierendes Hydrolysat dann auf Raumtemperatur abgekühlt, gerührt, einer Zugabe von Ethanol als Stabilisator in einer Menge von 2 % eines Gesamtvolumens des resultierenden Hydrolysats unterzogen wird, wobei anschließend das verbleibende Wasser zugesetzt wird, das resultierende Hydrolysat erneut gerührt wird, 60 Minuten lang absetzen gelassen wird, um ein Honigkonzentrat herzustellen, das anschließend filtriert, in Behälter gegossen und bei 110 °C autoklaviert wird.

## Revendications

1. Procédé de production d'un concentré de miel, comprenant une étape préparatoire comportant l'ajout de l'eau distillée et le chauffage d'homogénat, la réalisation d'une hydrolyse sous agitation continue et l'évaluation de la qualité d'un hydrolysat ainsi produit, **caractérisé en ce que** le miel est exposé à l'ajout de 80% d'eau purifiée par rapport à un volume total d'eau à ajouter, la température augmente jusqu'à 35-40°C et la valeur de pH de l'homogénat est ajustée jusqu'à 6,0, ensuite la température augmente davantage sous agitation intensive et un pH = 6,0 pour obtenir une température de 45°C, la valeur de pH est ajustée toutes les 10 minutes pendant une période allant de 50 à 60 minutes afin d'obtenir une valeur de pH stabilisée dans une plage allant de 4,5 à 5,0, ensuite un hydrolysat résultant est refroidi jusqu'à la température ambiante, exposé à une agitation, soumis à l'ajout d'éthanol utilisé comme stabilisant en une quantité de 2% d'un volume total de l'hydrolysat résultant, l'eau restante est par la suite ajoutée, l'hydrolysat résultant est à nouveau agité, laissé déposer pendant 60 minutes pour produire un concentré de miel qui est par la suite filtré, versé dans des récipients et traité à l'autoclave à 110°C.
